# EUROPEAN PATENT APPLICATION

(11) **EP 4 756 823 A2**
(43) Date of publication of application: **10.06.2026**
(21) Application number: 26172486.8
(22) Date of filing: 09.11.2020
(51) Int. Cl.: G16H 20/30

(54) **STIMULATION DEVICES AND SYSTEMS**

(30) Priority: 08.11.2019 US 201962933178 P
(62) Divisional of application: 20885194.9
(71) Applicant: United Therapeutics Corp., Silver Spring, MD 20910 (US)
(72) Inventor: TRACEY, Kevin, Old Greenwich, 06870 (US); LATHAN, Corinna, Silver Spring, 20901 (US); HAWKINS, Trevor, Alamo, 94507 (US)
(74) Representative: Studio Torta S.p.A.

(57) **Abstract**

Described herein are noninvasive electrical stimulation devices, systems and methods for stimulation of the Vagus nerve through its auricular branch to provide beneficial physiological responses in subjects, including alleviation, mitigation or elimination of symptoms of various disorders, including metabolic and inflammatory disorders.

## Description

### CROSS REFERENCE TO RELATED APPLICATION(S)

This application is a non-provisional patent application claiming the benefit of the filing date of U.S. Provisional Patent Application Serial No. 62/933,178, filed November 8, 2019, and titled "Stimulation Devices, Systems, and Methods" which is hereby incorporated by reference.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BACKGROUND

Technological approaches to healthcare and treatment of diseases have made countless leaps and bounds over the past century. These include everything from highly sensitive molecular diagnostics, to fundamentally new approaches to pharmacological treatment of varied and complex diseases and disorders. Many of these treatments have been accompanied by myriad unintended consequences, including harmful side effects, addictiveness, and significant cost.

This has led many researchers to seek approaches for the treatment or management of disorders that use or augment the body's own innate machinery in correcting a wide variety of disorders or conditions. Some of these approaches aim to rebalance the biochemical operation of the body through administration of certain supplements, such as increased doses of certain vitamins and minerals, supplementation and rebalancing of the body's microbial flora (or microbiome), or even through adjustments to basic nutrition.

In other cases, body's systems can be spurred into action through application of external stimuli to tissues and/or organs within the body in an attempt to get those organs and/or tissues to engage in a beneficial process or cascade, either directly or through activation of other tissues or organs. Examples of these approaches include electrical and mechanical stimulations of different tissue types in order to elicit a desired physiological response, such as metabolic control or modulation of blood glucose level.

Many researchers have identified the Vagus nerve as a potential avenue for addressing many different disorders. The Vagus nerve, also termed the pneumogastric nerve, is the tenth cranial nerve or CN X, and interfaces with the parasympathetic control of the heart, lungs, and digestive tract. Because of its role as a nexus of control of bodily functions, the activity of the Vagus nerve has been implicated in a wide range of physiological functions, which highlights its potential for diagnostic and/or therapeutic applications. By imparting electrical stimulation devices directly upon the Vagus nerve, physicians have been able to show some progress in alleviating symptoms of some disorders. To date, this has primarily focused on addressing seizure disorders, depression, tonal tinnitus and even weight loss.

Currently, stimulation of the Vagus nerve is done primary through implantation of stimulation devices underneath skin of a subject. Such approach requires surgery, is cumbersome, and runs a risk of infection. External Vagus nerve stimulation devices require manual stimulation by contacting the devices with the subject's skin. These external Vagus nerve stimulation devices can not precisely stimulate the Vagus nerve due to hinderance of subject's skin and muscle. Also, if the subject erroneously places the device too close to his or her heart, the stimulation can trigger severe side effects such as cardiac arrest. Additionally, the electrical stimulation delivered by devices currently available is not personalized and not based on continuously changes in the subject's physiology and needs for the Vagus nerve stimulation.

Therefore, there remains a pressing need for devices, systems, and methods to stimulate the Vagus nerve with precision and efficacy while simultaneously reduce the risks and severity of the side effects. Furthermore, there remains a need for the Vagus nerve stimulation devices, systems, and methods to be easily accessible and adaptable to tailor to the lifestyle of the subject. Lastly, there remains a need for the Vagus nerve stimulation devices, systems, and methods to deliver the electrical stimulation, where the parameters of the electrical stimulation can be personalized and modified in real-time.

### SUMMARY

Described herein, in some embodiments, is a method of producing a beneficial physiological response in a subject, comprising a stimulating step of stimulating a nucleus tractus solitarius in the subject by applying an electrical stimulus to an auricular branch of the Vagus nerve in the subject, wherein the electrical stimulus comprises one or more of a voltage, a current, a waveform, a frequency, an amplitude, a duration and a periodicity selected to produce the beneficial physiological effect in the subject. In some embodiments, the electrical stimulus comprises one or more of: an applied current of between about 1 microAmp and about 1000 microAmps; a waveform selected from sinusoidal wave, square wave, and combinations thereof; a frequency of between about 1Hz and about 100 KHz; a duration of between about 30 seconds and 1 hour; and a periodicity of between about 60 times per minute to about once per week. In some embodiments, the method comprises measuring one or more physiological properties of the subject following the stimulating step. In some cases, the method comprises measuring one or more physiological properties of the subject prior to the stimulating step. In some cases, the physiological properties measured by the method described herein are selected from heart rate, heart rate variability, respiratory rate, pulse oxygen, blood pressure, total peripheral resistance, tonometric radial artery waveforms, cardiac output, galvanic skin resistance, bowel sounds, body temperature, levels of salivary cortisol, sleep stage measures, rapid eye movements during sleep, eye movements, pupil dilation, EEG waveforms, brainstem evoked responses to optic or auditory stimulation, serum or urine levels of one or more biomarkers such as cytokine and chemokine levels (including, for example, IL-1β, IL-1ra, IL-2, IL-4, IL-5, IL-6, IL-7, IL-8, IL-9, IL-10, IL-12(p70), IL-13, IL-15, IL-17, Eotaxin, Basic FGF, G-CSF, GM-CSF, IFN-γ, IP-10, MCP-1, MIP-1α, PDGF-BB, MIP-1β, RANTES, TNF-β, VEGF, IL-1α, IL-2Rα, IL-3, IL-12(p40), IL-16, IL-18, CTACK, GRO-α, HGF, IFN-α2, LIF, MCP-3, M-CSF, MIF, MIG, β-NGF, SCF, SCGF-β, SDF-1α, TNF-α, TRAIL), CRP, reproductive hormones, thyroid hormones, adrenal hormones, pituitary hormones, 5-HIAA, 17-OH progesterone, 17-hydroxycorticosteroids, 17-ketosteroids, 24-hour urinary aldosterone excretion rate, 25-OH vitamin D, Adrenocorticotropic hormone (ACTH), ACTH stimulation test, ACTH suppression test, ADH, Aldosterone, Calcitonin, Catecholamines - blood, Catecholamines - urine, Cortisol level, Cortisol - urine, DHEA-sulfate, Follicle stimulating hormone (FSH), Growth hormone, HCG (qualitative - blood), HCG (qualitative - urine), HCG (quantitative), Luteinizing hormone (LH), LH response to GnRH, Parathormone, Prolactin, PTH-related peptide, Renin, T3RU test, Secretin stimulation test, Serotonin, T3, T4, Testosterone, Thyroid stimulating hormone (TSH), Albumin, Alkaline Phosphatase, Alanine Aminotransferase (ALT), Aspartate Aminotransferase (AST), Bilirubin (total and direct), Blood Glucose, Blood Urea Nitrogen, Calcium (Ca) in Blood, Carbon Dioxide (Bicarbonate), Chloride (Cl), Cholesterol and Triglycerides Tests, Creatinine and Creatinine Clearance, Gamma-Glutamyl Transferase (GGT), Lactate Dehydrogenase, Phosphate in Blood, Potassium (K) in Blood, Sodium (Na) in Blood, Total Serum Protein, and Uric Acid in Blood.

Described herein, in some embodiments, is an auricular nerve stimulation system, comprising: an auricular stimulation device comprising at least one first and at least one second electrode positioned spaced apart from each other and configured to contact a subject to provide an electrical stimulation to an auricular branch of the subject's Vagus nerve, wherein at least one electrode is configured to be in electrical contact with a surface of a subject's ear; a controller, coupled to the stimulation device and configured to control and/or modulate application of electrical stimulation delivered by the device in response to one or more input parameters; and a monitoring system, coupled to the controller for providing an assessment of the subject, the assessment providing the one or more input parameters to the controller. In some cases, the monitoring system comprises a system for measuring one or more physiological properties of the subject. In some embodiments, the stimulation system further comprises a stimulation normalization system for ensuring a consistent stimulation is delivered to different subjects or the same subject at different times. In some cases, the stimulation system measures an impedance between the first and second electrodes when positioned in contact with the subject, and modulates stimulation based upon said impedance. In some embodiments, the first and second electrodes are both configured to contact a cymba concha of the subject's ear. In some cases, the second electrode is positioned in electrical contact with a surface of the subject at a location distal from the first electrode. In some embodiments, the first electrode is in electrical contact with a cymba concha of a first ear of the subject and the second electrode is in electrical contact with a cymba concha of a second ear of the subject. In some instances, the stimulation system as described herein can measure or modulate one or more physiological properties are selected from heart rate, heart rate variability, respiratory rate, pulse oxygen, blood pressure, total peripheral resistance, tonometric radial artery waveforms, cardiac output, galvanic skin resistance, bowel sounds, body temperature, levels of salivary cortisol, sleep stage measures, rapid eye movements during sleep (REM) , eye movements, pupil dilation, EEG waveforms, brainstem evoked responses to optic or auditory stimulation, serum or urine levels of one or more biomarkers such as cytokine and chemokine levels (including, for example, IL-1β, IL-1ra, IL-2, IL-4, IL-5, IL-6, IL-7, IL-8, IL-9, IL-10, IL-12(p70), IL-13, IL-15, IL-17, Eotaxin, Basic FGF, G-CSF, GM-CSF, IFN-γ, IP-10, MCP-1, MIP-1α, PDGF-BB, MIP-1β, RANTES, TNF-β, VEGF, IL-1α, IL-2Rα, IL-3, IL-12(p40), IL-16, IL-18, CTACK, GRO-α, HGF, IFN-α2, LIF, MCP-3, M-CSF, MIF, MIG, β-NGF, SCF, SCGF-β, SDF-1α, TNF-α, TRAIL), CRP, reproductive hormones, thyroid hormones, adrenal hormones, pituitary hormones, 5-HIAA, 17-OH progesterone, 17-hydroxycorticosteroids, 17-ketosteroids, 24-hour urinary aldosterone excretion rate, 25-OH vitamin D, Adrenocorticotropic hormone (ACTH), ACTH stimulation test, ACTH suppression test, ADH, Aldosterone, Calcitonin, Catecholamines - blood, Catecholamines - urine, Cortisol level, Cortisol - urine, DHEA-sulfate, Follicle stimulating hormone (FSH), Growth hormone, HCG (qualitative - blood), HCG (qualitative - urine), HCG (quantitative), Luteinizing hormone (LH), LH response to GnRH, Parathormone, Prolactin, PTH-related peptide, Renin, T3RU test, Secretin stimulation test, Serotonin, T3, T4, Testosterone, Thyroid stimulating hormone (TSH), Albumin, Alkaline Phosphatase, Alanine Aminotransferase (ALT), Aspartate Aminotransferase (AST), Bilirubin (total and direct), Blood Glucose, Blood Urea Nitrogen, Calcium (Ca) in Blood, Carbon Dioxide (Bicarbonate), Chloride (Cl), Cholesterol and Triglycerides Tests, Creatinine and Creatinine Clearance, Gamma-Glutamyl Transferase (GGT), Lactate Dehydrogenase, Phosphate in Blood, Potassium (K) in Blood, Sodium (Na) in Blood, Total Serum Protein, and Uric Acid in Blood. In some cases, the monitoring system comprises a user input system for receiving feedback from the subject. In some alternatives, the monitoring system comprises an application on a smart device, that is coupled to the controller. In some cases, the application of the stimulation system comprises programming that poses a plurality of questions to the subject, and based upon answers provided by the subject, provides one or more input parameters to the controller. In some embodiments, the stimulation system further comprises a learning engine coupled to one or more of the monitoring systems and the controller, wherein the learning engine is programmed to aggregate assessment information and input parameters and determine one or more optimal input parameters or assessment criteria for auricular nerve stimulation.

Described herein, in some embodiments, is a nerve stimulation optimization system, comprising: a nerve stimulation device for delivering an electrical stimulation to an auricular branch of a Vagus nerve of a subject; an assessment device and controller for modulating the electrical stimulation delivered to the subject and assessing one or more parameters from the subject prior to and in response to electrical stimulation; and a processor coupled to the assessment device and the controller for receiving one or more of assessment data received by the assessment device and/or control data from the controller, and for determining an optimal set of control parameters for stimulating the auricular branch of the Vagus nerve in the subject in order to elicit one or more beneficial physiological responses in the subject.

Described herein, in some embodiment, is a therapeutic system, comprising: a housing structure configured to be positioned over, adjacent to, or within an ear of a subject; an auricular stimulation device integrated into the housing structure, comprising, comprising at least a first and a second electrode positioned opposite each other and configured to be positioned upon an inner and outer surface of the subjects ear when the housing structure is positioned over, within, or adjacent to the ear of the subject, so as to deliver electrical stimulation to an auricular branch of Vagus nerve of the subject; a controller, coupled to the stimulation device and configured to control and/or modulate application of electrical stimulation delivered by the device in response to one or more input parameters; and an audio delivery system integrated into the housing and positioned to direct audio signals to the subject's ear when the housing structure is placed over, within, or adjacent to the subject's ear. The therapeutic system can additionally comprise a controller that comprises audio content, said controller delivers the audio content to the audio delivery system. In some cases, the audio content delivered by the controller to the audio delivery system is selected to augment or increase a physiological impact of the electrical stimulation delivered by the stimulation device.

Described herein, in some embodiments, is a therapeutic method, comprising simultaneously delivering to a subject's ear an electrical stimulation to an auricular branch of subject's Vagus nerve in the ear and an audio signal, wherein each of the electrical stimulation and the audio signal are selected to provide a beneficial physiological response. In some embodiments, the therapeutic method comprises using the Vagus nerve stimulation devices, systems, and methods as described in the present disclosure.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments.
**FIG. 1** provides a schematic illustration of an overall stimulation system architecture as described herein.
**FIG. 2** provides a schematic illustration of an example of a stimulation device configuration as described herein.
**FIG. 3** shows a non-limiting example of a computing device for implementation of the Vagus nerve stimulation devices, methods, and systems as described herein. The device can have one or more processors, memory, storage, and a network interface.
**FIG. 4** shows a non-limiting example of a web/mobile application provision system for implementation of the Vagus nerve stimulation devices, methods, and systems as described herein. In this case, the system provides browser-based and/or native mobile user interfaces.
**FIG. 5** shows a non-limiting example of a cloud-based web/mobile application provision system for implementation of the Vagus nerve stimulation devices, methods, and systems as described herein. In this instance, the system comprises an elastically load balanced, auto-scaling web server and application server resources as well synchronously replicated databases.

### DETAILED DESCRIPTION

While preferred embodiments of the present disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein can be employed in practicing the disclosure. It is intended that the following claims define the scope of the disclosure and that methods and structures within the scope of these claims and their equivalents be covered thereby.

As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising."

The term "about" or "approximately" means within an acceptable error range for the particular value as determined by one of ordinary skill in the art, which will depend in part on how the value is measured or determined, e.g., the limitations of the measurement system. For example, "about" can mean within 1 or more than 1 standard deviation, per the practice in the given value. Where particular values are described in the application and claims, unless otherwise stated the term "about" should be assumed to mean an acceptable error range for the particular value. As used herein, the term "about" in some cases refers to an amount that is approximately the stated amount. As used herein, the term "about" refers to an amount that is near the stated amount by 10%, 5%, or 1%, including increments therein. As used herein, the term "about" in reference to a percentage refers to an amount that is greater or less the stated percentage by 10%, 5%, or 1%, including increments therein.

As used herein, the phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together.

Vagus nerve stimulation has shown a potential as an avenue for treating or managing a wide variety of health and wellness issues in the human body. For example, research has demonstrated that Vagus nerve stimulation can modulate cytokine production in alleviating symptoms of inflammatory diseases, such as rheumatoid arthritis (Koopman, et al., PNAS (2016) 113:8284-8289. Likewise, Vagus nerve stimulation has been implicated in controlling addictive impulses, e.g., in cocaine addiction (Childs et al., Learning and Memory (2016) 25:35-42), as well as reducing opiate withdrawal symptoms in addicted subjects (Miranda et al. Am. J. Drug and Alc. Abuse (2018) 44(1):56-63). Despite the promise of Vagus nerve stimulation as a therapeutic avenue for a host of different health and wellness issues, the relative invasiveness, the potential for non-targeted effects resulting from that stimulation or ineffectively directed stimulation has made advancement in the space challenging.

Described herein are methods, systems, devices and applications for non-invasive electrical stimulation of the auricular branch of the Vagus nerve and its related neural networks. The Vagus nerve operates as a signaling pathway between organs and systems within the body, and the nucleus tractus solitarius (NTS) within the brain stem. Sensory signals are transmitted from the body to the NTS via the Vagus nerve from the body's organs, e.g., the digestive tract, the diaphragm, the larynx, etc. The NTS then transmits responsive signals through the Vagus nerve to these systems to regulate their function in response to the sensory inputs. External stimulation of this neural pathway, thus, would be expected to provide signals in both directions, e.g., to both the NTS and the organs or systems themselves, which can lead to unforeseen and unregulated responses.

Unlike other aspects of the Vagus nerve, the auricular branch of the Vagus nerve does not appear to connect the NTS to any substantive organs or systems in the ear and appears to be an evolutionary artifact. Accordingly, stimulation of this neural pathway can provide advantages over general Vagus nerve stimulation, as it seemingly provides direct stimulation access to control the Vagus nerve by sending only afferent signals to the NTS, which can provide an intermediary between stimulation and the major organ systems in which an ultimate response is sought. Accordingly, provided herein are methods, systems, devices and applications for stimulating the auricular branch of the Vagus nerve of a subject to generate a beneficial physiological response in the subject. In some cases, stimulation of the auricular branch of the Vagus nerve stimulates a response in the NTS that results in a beneficial physiological response within the subject.

Also described herein, in some embodiments, are the Vagus nerve stimulation devices, methods, and systems that are subjected to analysis of computation and machine learning based on data, feedback, and inputs of the subjects or users of the Vagus nerve stimulation devices, methods, and systems. In some cases, the analysis can be performed by the learning engine described herein. In some embodiments, the analysis of data, feedback, and inputs of the subjects or users of the Vagus nerve stimulation devices, methods, and systems at least partially determine the parameters or regiments of the electrical stimulation delivered to the subjects or users. In some embodiments, the analysis of data, feedback, and inputs of the subjects or users of the Vagus nerve stimulation devices, methods, and systems is utilized to deliver personalized electrical stimulation to the subjects or users. In some embodiments, the analysis of data, feedback, and inputs of the subjects or users of the Vagus nerve stimulation devices, methods, and systems is utilized to deliver personalized electrical stimulation to the subjects or users. In some embodiments, the analysis of data, feedback, and inputs of the subjects or users of the Vagus nerve stimulation devices, methods, and systems is utilized to modify and deliver personalized electrical stimulation to the subjects or users over the entire duration of the subjects or users using the Vagus nerve stimulation devices, methods, and systems described herein.

### I. Stimulation

In accordance with this disclosure, stimulation employs electrical stimulation of the auricular branch of the Vagus nerve, which, in many cases, involves the direct application of electrical energy to a portion of the ear of the subject, such as the cymba concha, or other portions of the exterior ear structure. In some cases, the electrical stimulation is provided so as to elicit a desired physiological response in the subject. In some cases, electrical stimulation can be selected and/or modified in any one or more of a number of characteristics in order to selectively elicit one or more desired physiological responses in the subject. By way of example, the electrical stimulation can provide one or more of a selected current, frequency, pulse width, waveform, duration, and periodicity, so as to provide a desired physiological response in the subject to which it is being applied. By way of example, with respect to the characteristics of the applied electrical energy, one can select one or more such characteristics as set forth below.

In some cases, the applied electrical energy can provide an electrical current of from about 1 microAmp to about 5 milliAmps. In some cases, the applied current can be from about 50 to about 2 milliAmps. In other cases, the applied current can be between about 100 and about 2000 microAmps. In other cases, the applied current can be between about 200 and about 500 microAmps, between about 400 and 800 microAmps, and between about 300 and 900 microAmps. In some cases, the applied current can be about at least about 50 microAmps, at least about 100 microAmps, at least about 200 microAmps, 300 microAmps, at least about 400 microAmps, at least about 500 microAmps, at least about 600 microAmps, at least about 700 microAmps, at least about 700 microAmps, at least about 800 microAmps, at least about 900 microAmps, at least about 1000 microAmps, at least about 1100 microAmps, at least about 1200 microAmps, at least about 1300 microAmps, at least about 1400 microAmps, at least about 1500 microAmps, at least about 1600 microAmps, at least about 1700 microAmps, at least about 1800 microAmps, at least about 1900 microAmps, at least about 2000 microAmps, at least about 2100 microAmps at least about 2200 microAmps, at least about 2300 microAmps, at least about 2400 microAmps, at least about 2500 microAmps, or more. In some cases, the applied current can be between about 50 microAmps and about 500 microAmps. In other cases, the applied current can be between about 500 microAmps and about 1000 microAmps. In other cases, the applied current can be between about 1000 microAmps and 1500 microAmps. In certain cases, the applied current can be between about 1500 microAmps and about 2000 microAmps. In some cases, the applied current can be between about 2000 microAmps and about 2500 microAmps. In still other cases, the applied current can be between about 2500 microAmps and 5 milliAmps.

In some cases, a given application/stimulation can provide a varied current level, e.g., where the current level varies during application. In such cases, the current can vary throughout the ranges provided above. For example, in some cases, an applied current can vary during application from a low point between about 10 and 500 microAmps to a high point of from about 200 to about 2000 microAmps. As will be understood, this variation can start at the low end and gradually increase during application to the high point at the conclusion. In some cases, an inverted variation can be employed, e.g., starting high and finishing low. In still other cases, a varied profile can be configured that varies current in increasing and decreasing levels during the application according to any desired or selected current variation profile.

In some cases, controlled current is preferred in the application of the electrical energy, as it can address differences in impedance in how it is applied in any given subject. For example, in some cases, the site of application, e.g., the ear of a subject, can represent widely varying physical characteristics in terms of electrical coupling, e.g., as a result of different surface characteristics, such as hair, skin oil, moisture and salt level, which can yield widely varying impedance levels. As such, where desiring to apply a consistent electrical stimulation among different subjects (or the same subject at different times), applying a selected current provides an approach to normalizing electrical stimulation regardless of impedance. Alternatively, one can measure impedance at the time of application, and based upon the measured impedance, selecting the current, as described above.

In some cases, one can select the frequency of the applied electrical energy. In particular, in some cases, the applied electrical energy can have a frequency of between about 1 Hz and about 100,000 Hz. In some cases, the electrical energy is applied at a frequency of between 1 and about 10,000 Hz, between about 1 and 5000 Hz, between about 1 and 1000 Hz, between about 1 and about 500 Hz, between about 1 and about 100 Hz. In some cases, the applied electrical energy has a frequency of between about 10 and about 50Hz, with some cases applying an electrical energy at a frequency of about 10 Hz, 20, Hz, 30 Hz, or a frequency within this range, e.g., 10-30 Hz).

In some cases, the pulse width of the applied electrical energy can be between about 1 and about 999 milliseconds. In some cases, the pulse width can be between about 10 and about 500 milliseconds, while in other cases, the pulse width can be between about 10 and about 200 milliseconds. In other cases, the pulse width can be between about 1 and about 50 milliseconds, or between about 1 and about 25 milliseconds, or in other cases between about 25 and about 50 milliseconds. In still other cases, the pulse width can be between about 50 and about 100 milliseconds, between about 50 and 75 milliseconds or between about 75 and about 100 milliseconds.

The duration of the applied electrical energy stimulation may, in some cases, be between about 30 seconds and 1 hour. In some cases, the energy can be applied for about 30 seconds or longer, about 1 minute or longer, about 2 minutes or longer, about 3 minutes or longer, about 4 minutes or longer, about 5 minutes or longer, about 6 minutes or longer, about 7 minutes or longer, about 8 minutes or longer, about 9 minutes or longer, about 10 minutes or longer, about 15 minutes or longer, about 20 minutes or longer, about 30 minutes or longer, about 40 minutes or longer, about 50 minutes or longer, or even about 60 minutes, or longer. In some cases, the applied energy is for a duration of from about 1 minute to about an hour, from about 2 minutes to about 30 minutes, from about 3 minutes to about 10 minutes, or in some cases about 5 minutes, plus or minus 2 minutes, e.g., from 3-7 minutes.

The number of applications can likewise be selected to provide a desired result. In some cases, a single application can provide a desired result. In other cases, multiple applications can be desired, for example, from once per week, or once per day, to once per hour, to multiple applications per hour, e.g., from about 1 to about 60 applications per hour during a treatment regimen. In some cases, from 1 to 4 treatments can be applied per day of treatment, with treatment days being once per month, one day per week, or in some cases, 2, 3, 4 or 5 days per week, or in some cases, daily.

In some cases, one can control the waveform of the applied electrical energy in whole or in part to achieve a desired physiological response. For example, in some cases, a waveform can constitute a sinusoidal waveform, while in other cases, it can be characterized by a square waveform. In some cases, a waveform can be selected that, in part, helps provide the desired physiological response in the subject to which the energy is applied. Such waveforms can generally be characterized by any form from sinusoidal to square.

### II. Beneficial Physiological Response

### Overall

As noted above, one can apply the electrical energy to stimulate the auricular branch of the Vagus nerve in a subject to elicit a desired physiological response in the subject, by application of electrical energy that possesses one or more of the above described characteristics. As set forth herein, in order to achieve any of the variety desired physiological responses, one can modify one or more of the above described stimulation characteristics during one or more treatment regimens. For example, one can vary one or more of the electrical characteristics, such as current, frequency, waveform, etc., over the course of a single application, or over the course of multiple different applications, in order to achieve the desired physiological response(s).

The achievement of a desired physiological response through electrical stimulation of the auricular branch of the Vagus nerve of a subject can take any of a variety of different forms. In some cases, this is a result of stimulating the NTS to signal a physiological response in one or more other organs or systems within the subject's body. Desired physiological responses that can be driven by the NTS as a result of stimulation can include, for example, broad based or specific therapeutic results, including, for example, anti-inflammatory responses, such as system wide or organ specific changes in cytokine levels, altered metabolic responses, such as changes in blood sugar control within the subject, e.g., resulting in lowered or increased blood glucose, as well as controlling the blood or tissue levels of hormones, metabolites, neurotransmitters, cytokines, electrolytes, vitamins, nutrients, lipids, proteins, and the like.

Modulation of these physiological metrics can have broad applications in a wide range of different disorders. For example, eliciting certain physiological responses can have broad application in a range of disorders that have a significant inflammatory component in their pathogenesis, including: central nervous system inflammation disorders (such as multiple sclerosis (MS), amyotrophic lateral sclerosis (ALS), encephalitis, myelitis, and meningitis); peripheral nervous system inflammation disorders (such as neuritis); optical inflammation disorders (such as dacryoadenitis, scleritis, episcleritis, keratitis, iritis), aural inflammation disorders (such as otitis), cardiovascular inflammation disorders (such as carditis, endocarditis, myocarditis, pericarditis, vasculitis, arteritis, phlebitis and capillaritis); respiratory inflammation disorders (such as sinusitis, rhinitis, pharyngitis, epiglottitis, laryngitis, tracheitis, bronchitis, bronchiolitis, pneumonitis, pneumonia, and pleurisy); digestive system inflammation disorders (such as inflammatory bowel disease, IBD), irritable bowel syndrome (types C, D and C/D), stomatitis, gingivitis, glossitis, tonsillitis, sialadenitis, parotitis, cheilitis, pulpitis, gnathitis, oesophagitis, gastritis, gastroenteritris, enteritis, colitis, pancolitis, appendicitis, cryptitis, hepatitis, viral hepatitis, alcoholic hepatitis, non-alcoholic steatohepatitis (NASH), autoimmune hepatitis, non-alcoholic fatty liver disease (NAFLD), cholexcystitis, and pancreatitis); integumentary system inflammation disorders (such as dermatitis and mastitis), skeletal system inflammation disorders (such as arthritis, myositis, synovitis, tenosynovitis and bursitis); urinary system inflammation disorders (such as nephritis, ureteritis, cystitis, and urethritis); reproductive system inflammation disorders (such as oophoritis, salpingitis, metritis, endometritis, myometritis, parametritis, cervicitis, vaginitis, vulvitis, orchitis, epididymitis, prostatitis, vesiculitis, balanitis, and posthitis); endocrine system inflammation disorders (such as insulitis, hypophysitis, thyroiditis, parathyroiditis, and adrenalitis); and lymphatic system inflammation disorders (such as lymphangitis and lymphadenitis).

**In** alternative or additional cases, the stimulation methods and systems described herein can provide more generalized physiological responses, that can impact general health and wellness, including, for example, pain management, improved sleep, reduced anxiety, weight loss and diet control, smoking cessation, improved cognition, and mitigation of digestive issues.

As alluded to above, by modulating one or more of the parameters of the electrical stimulation, it is expected that one can elicit different physiological responses in the subject. For example, by changing one or more of, for example, the frequency, current, duration, etc., of the electrical stimulation, one can elicit either or both of an anti-inflammatory response or a beneficial metabolic response. Thus, in order to achieve a desired physiological response can require application of electrical stimulation having selected electrical parameters. In some cases, one can ascertain the different physiological responses by modulating the electrical parameters of the stimulation while monitoring the subject for signals or metrics associated with the various physiological responses. By way of example, application of electrical stimulation through the cymba concha at 20 Hz, with a pulse width of 200 microseconds, using an applied current that is physically tolerated by the subject, e.g., a period of 5 minutes has been shown to reduce pain in subjects suffering from migraine headaches, as well as in those suffering from back pain, sciatica pain and inflammatory bowel disease, as well as providing improved cognitive functions (recall and learning), as well as improving recovery from soft tissue injury to the extremities.

### Metrics

**In** some cases, beneficial physiological responses within a subject as a result of electrical stimulation as described herein, can be identified and or previewed through a variety of biological markers or metrics. In some cases, such markers can be direct indicators of the beneficial physiological response, e.g., reduction in blood glucose, while in other cases, the metrics can alone, or in combination reflect physiological benefit as a result of increased or decreased biochemical activity, e.g., enhanced or reduced cytokine production which would in turn, result in changes in inflammatory responses. Accordingly, the physiological impacts of stimulation can include alterations in any one or more of the following biological metrics in the subject. Such metrics include for example, biometrics, such as heart rate, heart rate variability, respiratory rate, pulse oxygen, blood pressure, total peripheral resistance, tonometric radial artery waveforms, cardiac output, galvanic skin resistance, bowel sounds, body temperature, levels of salivary cortisol, sleep stage measures (REM, etc.), eye movements, pupil dilation, EEG waveforms, and brainstem evoked responses to optic or auditory stimulation.

Additionally, or alternatively, the physiological responses can be identified, indicated or previewed by responses in certain blood, serum or urine levels of one or more biomarkers such as cytokine and chemokine levels (including, for example, IL-1β, IL-1ra, IL-2, IL-4, IL-5, IL-6, IL-7, IL-8, IL-9, IL-10, IL-12(p70), IL-13, IL-15, IL-17, Eotaxin, Basic FGF, G-CSF, GM-CSF, IFN-γ, IP-10, MCP-1, MIP-1α, PDGF-BB, MIP-1β, RANTES, TNF-β, VEGF, IL-1α, IL-2Rα, IL-3, IL-12(p40), IL-16, IL-18, CTACK, GRO-α, HGF, IFN-α2, LIF, MCP-3, M-CSF, MIF, MIG, β-NGF, SCF, SCGF-β, SDF-1α, TNF-α, TRAIL), CRP, reproductive hormones, thyroid hormones, adrenal hormones, pituitary hormones, 5-HIAA, 17-OH progesterone, 17-hydroxycorticosteroids, 17-ketosteroids, 24-hour urinary aldosterone excretion rate, 25-OH vitamin D, Adrenocorticotropic hormone (ACTH), ACTH stimulation test, ACTH suppression test, ADH, Aldosterone, Calcitonin, Catecholamines - blood, Catecholamines - urine, Cortisol level, Cortisol - urine, DHEA-sulfate, Follicle stimulating hormone (FSH), Growth hormone, HCG (qualitative - blood), HCG (qualitative - urine), HCG (quantitative), Luteinizing hormone (LH), LH response to GnRH, Parathormone, Prolactin, PTH-related peptide, Renin, T3RU test, Secretin stimulation test, Serotonin, T3, T4, Testosterone, Thyroid stimulating hormone (TSH), Albumin, Alkaline Phosphatase, Alanine Aminotransferase (ALT), Aspartate Aminotransferase (AST), Bilirubin (total and direct), Blood Glucose, Blood Urea Nitrogen, calcium (Ca) in Blood, Carbon Dioxide (Bicarbonate), Chloride (Cl), Cholesterol and Triglycerides Tests, Creatinine and Creatinine Clearance, Gamma-Glutamyl Transferase (GGT), Lactate Dehydrogenase, Phosphate in Blood, potassium (K) in Blood, sodium (Na) in Blood, Total Serum Protein, and Uric Acid in Blood.

In some case, metrics of physiological responses can include subjective metrics from those being stimulated. Such metrics can include, for example, reduced anxiety, better sleep, calmness, happiness or reduced depression, or the like. Typically, such metrics can be identified, previewed or measured by direct reporting from the subject, e.g., in response to surveys, or through a companion app on a smart device.

As discussed in greater detail below, in some cases, the measured physiological responses can be used to inform the then current stimulation protocol or subsequent stimulation protocols. This feedback can provide guidance for the system in applying stimulation to a given subject, or, via the database and learning engine described below, provide feedback to modify stimulation to other subjects. By way of example, where a stimulation protocol in a given subject provides a suboptimal physiological response in that subject, as measured by the monitoring system, the system can be configured to modify the stimulation protocols, either during the current stimulation protocol, or in certain cases, in subsequent stimulation sessions, e.g., by providing increased or decreased current, altered frequency, altered waveforms, etc. in the stimulation protocol.

### III. Stimulation System

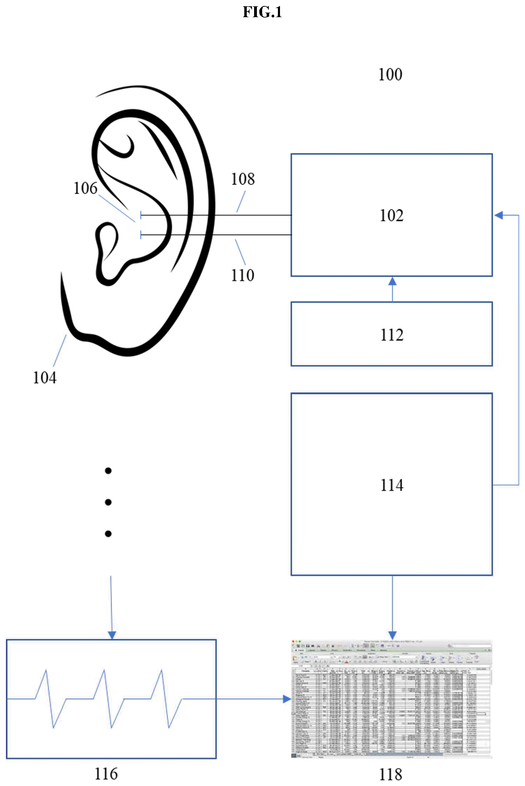

**FIG. 1** provides a schematic illustration of one example of a stimulation system as described herein. As shown, the system **100** includes a stimulation device **102,** that is positioned to provide electrical stimulation to the auricular branch of the Vagus nerve transcutaneously via the ear of the subject **104.** As shown in **FIG. 1****,** the stimulation device can position electrodes **108** and **110** into electrical contact with, for example, the cymba concha **106** of the subject's ear. The stimulation device includes or is coupled to a power source **112** that provides the requisite electrical energy for stimulation. Also coupled to the stimulation device is a controller device **114** or system for instructing the provision of electrical impulses to the subject through the stimulation device **102.** In some cases, the system also includes one or more monitoring devices or systems **116** for obtaining feedback on the physiological outcomes or responses, if any, following and/or resulting from one or more stimulation events. The monitoring device **116** can be in communication with a computer database **118** for storage of the physiological response data. Similarly, the controller can also be coupled to the database **118,** for storage of the stimulation parameter data that resulted in the physiological response data from the monitoring device **116.**

### Stimulation Device

As noted above, stimulation devices as used in the described methods will typically include at least a pair of electrodes that are configured to provide an electrical contact within the ear of the subject in order to deliver an electrical stimulus to the ear of the subject. The pair of electrodes can be positioned proximal to each other, so that both electrodes contact the cymba concha within the same ear of the subject, and which are sufficiently spaced apart to avoid electrical communication between the electrodes other than through the subject's cymba concha, e.g., to avoid shorting between the electrodes. Alternatively, or additionally, electrodes can be provided with an insulator sleeve or jacket to mitigate potential shorting either to the other electrode or through suboptimal current paths on the subject's skin surface. Alternatively, the electrodes can be positioned such that a single electrode is positioned against the cymba concha within the subject's ear, and the other electrode is positioned elsewhere on the subject, but so as to provide a current path through the cymba concha between the electrodes. In some cases, the second electrode can be positioned against the outer surface of the subject's ear so that the two electrodes are positioned on and biased against, or "clamping", against opposing surfaces of the subject's ear. In other cases, the second electrode can be positioned on a surface of the subject's skin that is distal to the positioning of the first electrode, but such that current is still able to flow between the electrodes. For example, in some cases, the second electrode can be positioned against the cymba concha of the opposing ear of the subject, such that electrical stimulation is applied to both ears of the subject with only a pair of electrodes.

As described herein, the Vagus nerve devices, systems, and methods can utilize the first and second electrodes to deliver electrical stimulation. In some cases, the first and second electrodes can both deliver the electrical stimulation. In some cases, one of the first or second electrodes can deliver the electrical stimulation. In some cases, the first electrodes and the second electrodes can deliver the electrical stimulation separately. In some cases, the first electrodes can deliver the electrical stimulation independent of the second electrodes. In some cases, the second electrodes can deliver the electrical stimulation independent of the first electrodes. In some cases, the parameters of the electrical stimulation delivered by the first or second electrodes can be modified to deliver personalized electrical stimulation. In some cases, the parameters of the electrical stimulation delivered by the first electrodes can be modified independent of the second electrodes. In some cases, the parameters of the electrical stimulation delivered by the second electrodes can be modified independent of the first electrodes.

The electrodes can be electrically coupled to a power source, for delivery of the electrical stimulation to the electrodes. The electrodes and power source can also be coupled to a controller unit that allows for a directed set of electrical parameters within the electrical impulses sent from the power source to the electrodes.

The electrodes, power source and controller can be housed within or mounted upon a wearable device, such as a headset, ear-phone, ear cuff, headphone or other similar structure, so as to position the electrodes against and in electrical contact with the surface of the exterior ear structure, such as the cymba concha. In some cases, the electrodes can be mounted upon a flexible mount, so as to bias the electrodes against the surface of the ear without providing discomfort to the subject. Examples, of electrode devices can include, for example, those described in Published International Patent Application No. WO 2019/028000 (the full disclosure of which is hereby incorporated herein by reference in its entirety for all purposes), which describes a clip mounted electrode set for positioning electrodes proximal to the ear.

In some cases, the electrodes can be provided within a combined device, such as within a set of audio headphones, ear-phones, ear-buds, or the like. In such cases, one can combine audio stimulation or entertainment during electrical stimulation

The electrodes can generally be structured in any of a variety of ways to ensure adequate contact and conductivity for the electrical impulses to be delivered to the subject. For example, in some cases, the electrodes can provide a hemispherical surface for contacting the surface of the ear directly and with reduced discomfort. Alternatively, conical electrodes can be provided in order to ensure targeted contact within the ear.

Electrode materials can include any of a variety of known conductive materials that are compatible with skin contact, including, for example, platinum, gold, stainless steel, aluminum, sintered steel, conductive polymers, carbon or metal infused plastics or ceramics, or the like. In other cases, non-conductive porous materials, such as polymer foams, can be provided over the tips of the electrodes, which can be saturated with a conductive medium, e.g., a solution or gel, that provides suitable electrical contact. Likewise, in many cases, a conductive medium can be used in conjunction with any of the aforementioned electrode configurations in order to ensure optimal electrical communication between the electrode and the surface of the subject's ear, or other skin location, as the case can be. In other cases, a conductive polymer tip can be placed over the electrode tip, where the polymer tip can include a hydrated, conductive polymer matrix. In some cases, the conductive polymer tips can be removable and disposable, in order to allow replacement with fresh conductive tips.

In some cases, the power supply is integrated into the same device as the electrodes, while in other cases, it can be separate, but electrically coupled by wire. In some cases, the power source can include a battery, such as a 9 volt or other battery that provides sufficient power to provide the electrical stimulation, in conjunction with appropriate circuitry for transmitting a desired electrical impulse. In some cases, the battery comprises a rechargeable battery, such as a lithium ion battery, or set of batteries that includes a charging port for coupling the device to a charging unit, e.g., a plug-in adapter, a USB or other port on a computer, or the like.

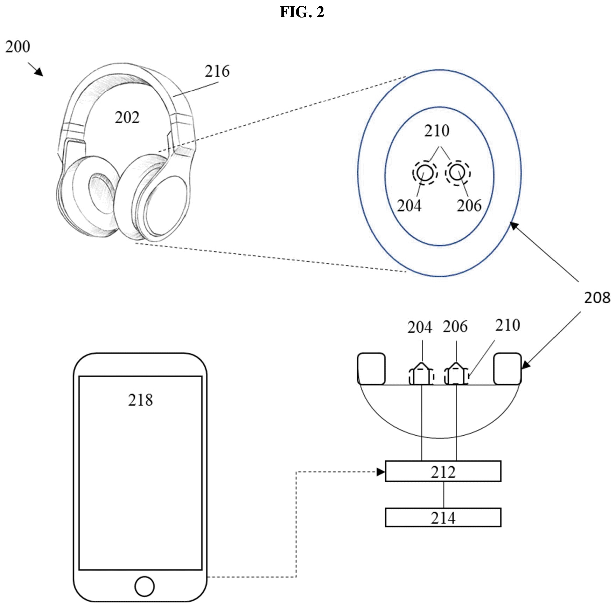

**FIG. 2** shows a schematic illustration of an example of a stimulation device as described herein. As shown, a device **200** is configured in a set of headphones **202,** where two electrodes **204** and **206** are provided disposed within one earpiece **208** of the headphones **202.** The electrodes **204** and **206** are positioned to seat against the cymba concha of the subject's ear when the ear piece **208** is seated over the subject's ear, as shown in the end on view of the earpiece **208.** Also shown is an optional insulator sleeve **210** provided around each electrode. The electrodes are coupled to the stimulation device **212** itself, e.g., the component that provides the stimulation profile to the electrodes, and a power source **214.** The stimulation device and power source can be positioned within the ear piece or within the headband **216** of the headphone device. Alternatively, these components can be included in a separate housing or case that is coupled to the headphones via an appropriate cable. As will be appreciated, the headphones are also capable of delivering audio to the subject, in the form of instructions, music, or other auditory signals as needed. As shown, the device is shown coupled to a smart phone **218,** which operates as a controller for the device. As noted elsewhere herein, the controller, whether a smart phone, or other mart device, can be coupled to the device directly, e.g., through physical wiring, or via wireless connection such as WIFI, Bluetooth, cellular or other wireless communication.

### Controllers

As noted above, delivery of electrical impulses from the power source to the electrodes for electrical stimulation within or upon the surface of the ear of a subject can be controlled by a controller component. As with the power source, the controller can be integrated into the electrode supporting device, or it can be separate and coupled to the power source. The controller can generally control the various parameters of the electrical impulses used for stimulation, including, e.g., the frequency, current, waveform, timing, duration, periodicity, etc. The controller can be operating from preprogrammed instructions, from direct user input into the device, or from an additional user interface device that allows a user to select one or more parameters to modify, or one or more preprogrammed sets of parameters for stimulation.

For example, a controller can deliver an electrical impulse to the electrodes in response to a selected input from a user, e.g., to address a particular issue for the user. In such cases, the electrical parameters delivered in such impulse and controlled by such controller can be tailored to provide the desired response. In some cases, an electrical impulse can be tailored to provide a baseline physiological response from which a user can choose to modify in order to optimize for their particular issue. For example, where a user is seeking to remedy a generalized issue or disorder, a first electrical impulse maybe provided. Following the first stimulation, the user can elect to modify the impulse, e.g., to increase or decrease one or more parameters or to shift the nature of the physiological response, e.g. targeting a slightly different response.

In some cases, the controller can additionally be coupled to a processor for storing instructions for the controller and/or for providing a user interface to the stimulation device. Such processor can include a directly coupled interface device that is hardwired to the device. In many cases, the processor can include a computer or smart device, such as a tablet, smart phone, smart watch, or similar device that is wirelessly connected to the stimulation device, e.g., through a Bluetooth or WIFI connection.

As noted above, the processor can provide a user input that allows a user to select a desired stimulation protocol. For example, a user who is managing a particular disorder or issue can select the protocol or program that is intended to target a physiological response to address that particular issue or disorder. In some cases, the programmed protocols can be obtained and uploaded to the processor from a database of protocols that have been identified as providing a given physiological response. In other cases, a user's own responses can be stored within the processor so that a user selects a protocol that as demonstrated efficacy for that user previously.

In some instances, the processor analyzes the data collected from the subject's or user's data of use, feedback, or input with machine learning methods to modified parameters for delivery of electrical stimulation. In some cases, the processor is the learning engine. In some cases, the processor is part of the learning engine. In some embodiments, the analysis performed by the processor is performed contemporaneously as the subject or the user using the Vagus nerve stimulation devices, systems, and methods as described herein. Exemplary modifications as produced by the analysis performed by the processor include modifications in the parameters, duration, or regiment of the electrical stimulation. In some cases, the modifications can include delivering electrical stimulation through one or both electrodes to one ear. In some cases, the modifications can include delivering electrical stimulation through one or both electrodes to both ears.

### Monitoring

Stimulation systems as described herein, can in some cases, include a monitoring system for detecting and/or measuring the responsive metrics in the subject prior to, during and/or following stimulation. Such monitoring systems can be configured to measure any one or more of the metrics described above, including both subjective metrics and the above described biomarkers and other biometrics.

In some cases, a monitoring device can include a user interface and input device that allows a subject to enter their subjective responses to a stimulation event. For example, a user input interface and device can include one or more inputs that allow the subject to input their relative levels of anxiety, calmness, cravings, fatigue, pain, or the like. Such inputs can include, for example, sliding scales of any one or more of these and other subjective metrics, where a user inputs, for example. A user can be asked to input their subjective metrics prior to, during, and or after a stimulation event. In some cases, the subjective inputs can include tests, such as for cognition, e.g., recall, perception, and the like, to assess general cognitive abilities. Such assessments can be incorporated into an associated app on a smart device, such as the DANA Brain app, which can measure speed and accuracy of responses to game like tests, for attention and processing speed of the subject.

In some cases, such monitoring device can the same smart device that operates as the controller element, which is then able to immediately aggregate response data with data related to the stimulation event, to provide an individual subject's response signature to that particular stimulation.

In some cases, the monitoring system can include additional separate or integrated devices for monitoring of other types of biometrics. For example, in some cases, a monitoring system can include a monitor of heart rate, a pulse oxygen, blood pressure, electrocardiogram, a body temperature, galvanic skin response, and the like. Such monitoring systems may, again, be integrated into a smart device worn against the skin or with an appropriate accessory device. By way of example, smart watches are generally configured to provide monitoring of a number of the foregoing metrics and can be at least a part of the overall monitoring system. For example, smart wearable devices, such as the Apple Watch, the Garmin VivoActive and Fenix series and other wearables include biometric monitoring, such as heart rate (including electrocardiograms, in the case of the Apple Watch), pulse-ox, and other parameters.

In some cases, the monitoring systems can be auxiliary systems, e.g., analytical systems for measuring biomarkers in blood or urine or transdermally, or intra-abdominally, which can then provide the relevant data before, during or after stimulation. These auxiliary systems can include external analytical systems, e.g., diagnostic detection systems, or they can again include systems carried and or worn by the subject. By way of example, in the case of applications for managing metabolic disorders, e.g., elevated blood glucose, a monitoring system can include a Continuous Glucose Monitor (CGM), such as the Libre Freestyle CGM available from Abbott Labs, the Dexcom G6 CGM available from Dexcom, Inc., and the Medtronic Guardian series of CGMs available from Medtronic, Inc. Such systems can provide continuous blood glucose monitoring data before, during and/or after stimulation. These and other systems can employ patches that are affixed to the subject and that either monitor analytes transdermally, or through a small inserted probe. Other systems can employ ingestible sensors, such as those available from Proteus Digital Health, which can provide biometric feedback before, during and/or after stimulation. These systems can measure any of a wide variety of physiological states within a subject, including temperature, blood pressure, system pH, e.g., stomach or gut pH, or levels of specific analytes in those systems.

In some cases, a monitoring system can alternatively or additionally include monitors of neural activity such as electroencephalograms (EEG) or electromyograms (EMG), and monitoring of electromagnetic fields associated with neural signal propagation, e.g., along neural pathways, such as the Vagus nerve, the Phrenic nerve, cranial and other nerves, as well as monitoring neural signals that terminate at various organ or other systems of the body.

### IV. Databases, Learning and Feedback

In some embodiments, the Vagus nerve stimulation devices, systems, and methods disclosed herein include one or more databases, or use of the same. In view of the disclosure provided herein, those of skill in the art will recognize that many databases are suitable for storage and retrieval of information related stimulation devices, systems, and methods as described herein. In various embodiments, suitable databases include, by way of non-limiting examples, relational databases, non-relational databases, object-oriented databases, object databases, entity-relationship model databases, associative databases, and XML databases. Further non-limiting examples include SQL, PostgreSQL, MySQL, Oracle, DB2, and Sybase. In some embodiments, a database is internet-based. In further embodiments, a database is web-based. In still further embodiments, a database is cloud computing-based. In a particular embodiment, a database is a distributed database. In other embodiments, a database is based on one or more local computer storage devices.

As noted previously, also provided herein are databases of electrical stimulation parameters and physiological responses that result therefrom. The electrical stimulation parameters include the above described parameters, as well as modulations of those parameters. Similarly, measured or identified physiological responses, such as any one or more of those described above, can similarly be stored in the databases and associated with the electrical parameters. Likewise, additional parameters related to the subjects themselves can be included within the database, e.g., height, weight, body mass index ("BMI"), health conditions, as well as any number of other subject specific parameters. In some cases, a subject's genetic signature can also inform the physiological response expected to stimulation, generally, or with respect to any particular intended physiological response. Such genetic signature can be derived from specific marker identification, e.g., from an exome or partial sequence, or it can be derived from whole genome, whole exome or other targeted sequencing of a subject's genome. Likewise, a particular expression profile, e.g., measured from sequenced/identified mRNA, can likewise dictate a particular response profile for a subject at a given time.

The databases can be built from user data obtained before, during and/or after the subjects' use of the stimulation devices, and can generally be cloud-based databases, in order to optimize storage as well as facilitate data analysis and processing from those databases. As noted above, in some cases, the data can be obtained from the above described monitoring devices or systems which can also be used in conjunction with the stimulation devices described herein, in order to provide feedback on physiological responses resulting from a stimulation event. As noted above, monitoring devices can include systems for monitoring biomarkers, e.g., through blood or urine derived biomarkers. Alternatively or additionally, the monitoring devices can be worn or adhered to the subject to measure, e.g., heart rate, respiration rate, bold pressure, pulse oxygen, galvanic skin response, blood glucose, etc., and can be configured in a smart watch format, as a sensing patch or pad that is connected wirelessly or otherwise to a monitor system or smart device. As with the controller element, such monitoring systems can be coupled to a processor, including a smart device or tablet, for recording the metrics in conjunction with an applied stimulation event. Such processors can then either directly, or through communication with an electronic storage system, e.g., cloud-based storage, store the metrics and parameters concurrently within the database.

In addition to the stimulation parameter data and the physiological data, the databases can also include subjects' specific data, including but not limited to age, sex, height, weight, body mass index (BMI), body fat percentage, blood pressure, medical history (history of disease, family medical history), current medications, genetic information, and the like.

In addition to providing an aggregation of stimulation and response data, the databases described herein can be used to support broad and deep learning about the effects of wide ranging variances in electrical parameters during stimulation , e.g., including but not limited to the stimulation parameters described above, on a wide range of different physiological responses, including but not limited to any one or more or all of the above described physiological metrics.

In particular, the systems described herein can additionally include a processing system, or learning engine, that includes artificial intelligence and machine learning algorithms and probabilistic modeling that can be applied to the substantial aggregation of both the wide-ranging stimulation parameters and the wide ranging physiological response data, in order to identify specific parameters or sets of parameters that can be applicable to specific physiological responses either for individual subjects or for varying types of subjects. For example, application of probabilistic models to large aggregations of the cause data (stimulation parameters) and effect data (physiological response metrics) derived from 1,2, 3, 4, 5, 10, 20, 30, 40, 50, 100, 200, 300, 400, 500, 1,000, 10,000, 20,000, 100,000, 500,000, 1,000,000 or more stimulation events on 1, 2, 3, 4, 5, 10, 20, 30, 40, 50, 100, 200, 300, 400, 500, 1,000, 5,000, 10,000, 15,000, 20,000, 25,000, 50,000, 100,000, 500,000, 1,000,000 or more subjects, can allow identification of stimulation parameters that are most likely to provide a desired beneficial physiological response in a subject in general, or in subjects of certain classes and types, e.g., having certain genotypes, certain medical conditions or histories, or the like. For example, such models can predict a greater likelihood of mood improvement in a subject with a first set of stimulation parameters. Similarly, a second set of stimulation parameters can be determined to likely provide a beneficial anti-inflammatory response in another subject. Moreover, a third set of stimulation parameters can be identified as likely to provide a beneficial metabolic response in a subject, e.g., reduce blood glucose levels.

The learning engines can include any one or more methods and approaches used in modeling large aggregations of cause and effect data and can take results from initial and subsequent analyses in order to learn and optimize parameters over time.

The learning can employ any one or more of a variety of practiced learning methods, including, for example, Artificial Intelligence (AI) methods, Hidden Markov methods and Deep Learning (multi-layered neural network) methods. Any of these methods can be implemented in the feedback structure presented by the learning system described herein. Examples of such leaning systems that coordinate multiple measured responses include, e.g., those described in Published U.S. Patent Application No. 2019-0050532A1, the full disclosure of which is incorporated herein by reference in its entirety for all purposes.

In some cases, the Vagus nerve stimulation devices, systems, and methods as described herein can comprise computer-implemented methods of supervised or unsupervised learning methods, including SVM, random forests, clustering algorithm (or software module), gradient boosting, logistic regression, and/or decision trees. The machine learning methods as described herein can improve parameters of the electrical stimulation. In some cases, the machine leaning methods can provide parameters or modification of parameters of the electrical stimulation personalized to the subject.

Supervised learning algorithms can be algorithms that rely on the use of a set of labeled, paired training data examples to infer the relationship between an input data and output data. Unsupervised learning algorithms can be algorithms used to draw inferences from training data sets to output data. Unsupervised learning algorithms can comprise cluster analysis, which can be used for exploratory data analysis to find hidden patterns or groupings in process data. One example of an unsupervised learning method can comprise principal component analysis. Principal component analysis can comprise reducing the dimensionality of one or more variables. The dimensionality of a given variables can be at least 1, 5, 10, 50, 100, 200, 300, 400, 500, 600, 700, 800, 900, 1000, 1100, 1200 1300, 1400, 1500, 1600, 1700, 1800, or greater. The dimensionality of a given variables can be at most 1800, 1600, 1500, 1400, 1300, 1200, 1100, 1000, 900, 800, 700, 600, 500, 400, 300, 200, 100, 50, 10 or less.

The computer-implemented methods can comprise statistical techniques. In some embodiments, statistical techniques can comprise linear regression, classification, resampling methods, subset selection, shrinkage, dimension reduction, nonlinear models, tree-based methods, support vector machines, unsupervised learning, or any combination thereof.

A linear regression can be a method to predict a target variable by fitting the best linear relationship between a dependent and independent variable. The best fit can mean that the sum of all distances between a shape and actual observations at each point is the least. Linear regression can comprise simple linear regression and multiple linear regression. A simple linear regression can use a single independent variable to predict a dependent variable. A multiple linear regression can use more than one independent variable to predict a dependent variable by fitting a best linear relationship.

A classification can be a data mining technique that assigns categories to a collection of data in order to achieve accurate predictions and analysis. Classification techniques can comprise logistic regression and discriminant analysis. Logistic regression can be used when a dependent variable is dichotomous (binary). Logistic regression can be used to discover and describe a relationship between one dependent binary variable and one or more nominal, ordinal, interval or ratio-level independent variables. A resampling can be a method comprising drawing repeated samples from original data samples. A resampling can not involve a utilization of a generic distribution tables in order to compute approximate probability values. A resampling can generate a unique sampling distribution on a basis of an actual data. In some embodiments, a resampling can use experimental methods, rather than analytical methods, to generate a unique sampling distribution. Resampling techniques can comprise bootstrapping and cross-validation. Bootstrapping can be performed by sampling with replacement from original data and take "not chosen" data points as test cases. Cross validation can be performed by split training data into a plurality of parts.

A subset selection can identify a subset of predictors related to a response. A subset selection can comprise best-subset selection, forward stepwise selection, backward stepwise selection, hybrid method, or any combination thereof. In some instances, shrinkage fits a model involving all predictors, but estimated coefficients are shrunken towards zero relative to the least squares estimates. This shrinkage can reduce variance. A shrinkage can comprise ridge regression and a lasso. A dimension reduction can reduce a problem of estimating n + 1 coefficients to a simpler problem of m + 1 coefficients, where m < n. It can be attained by computing n different linear combinations, or projections, of variables. Then these n projections are used as predictors to fit a linear regression model by least squares. Dimension reduction can comprise principal component regression and partial least squares. A principal component regression can be used to derive a low dimensional set of features from a large set of variables. A principal component used in a principal component regression can capture the most variance in data using linear combinations of data in subsequently orthogonal directions. The partial least squares can be a supervised alternative to principal component regression because partial least squares can make use of a response variable in order to identify new features.

A nonlinear regression can be a form of regression analysis in which observational data are modeled by a function which is a nonlinear combination of model parameters and depends on one or more independent variables. A nonlinear regression can comprise a step function, piecewise function, spline, generalized additive model, or any combination thereof.

Tree-based methods can be used for both regression and classification problems. Regression and classification problems can involve stratifying or segmenting the predictor space into a number of simple regions. Tree-based methods can comprise bagging, boosting, random forest, or any combination thereof. Bagging can decrease a variance of prediction by generating additional data for training from the original dataset using combinations with repetitions to produce multistep of the same carnality/size as original data. Boosting can calculate an output using several different models and then average a result using a weighted average approach. A random forest algorithm can draw random bootstrap samples of a training set. Support vector machines can be classification techniques. Support vector machines can comprise finding a hyperplane that best separates two classes of points with the maximum margin. Support vector machines can constrain an optimization problem such that a margin is maximized subject to a constraint that it perfectly classifies data.

Unsupervised methods can be methods to draw inferences from datasets comprising input data without labeled responses. Unsupervised methods can comprise clustering, principal component analysis, k-Mean clustering, hierarchical clustering, or any combination thereof.

### V. Computer Systems

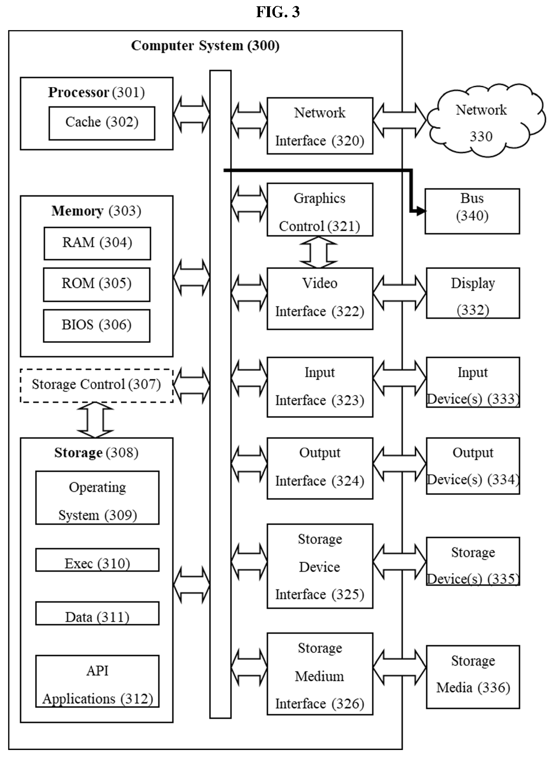

In some embodiments, the Vagus nerve stimulation devices, systems and methods as described herein comprise using of computer systems. In some cases, the computer systems can be used to store or analyze data generated from the subject's or user's use of the Vagus nerve stimulation devices. In some cases, the computer systems can at least partially be the learning engine. In some cases, the computer systems allow the subject to directly provide data, feedback, or input. In some embodiments, the computer systems can modify and provide the personalized parameters to the subject in real-time. Referring to **FIG. 3****,** a block diagram is shown depicting an exemplary machine that includes a computer system **300** (e.g., a processing or computing system) within which a set of instructions can execute for causing a device to perform or execute any one or more of the aspects and/or methodologies for static code scheduling of the present disclosure. The components in **FIG. 3** are examples only and do not limit the scope of use or functionality of any hardware, software, embedded logic component, or a combination of two or more such components implementing particular embodiments.

Computer system **300** can include one or more processors **301,** a memory **303**, and a storage **308** that communicate with each other, and with other components, via a bus **340.** The bus **340** can also link a display **332,** one or more input devices **333** (which may, for example, include a keypad, a keyboard, a mouse, a stylus, etc.), one or more output devices **334,** one or more storage devices **335,** and various tangible storage media **336.** All of these elements can interface directly or via one or more interfaces or adaptors to the bus **340.** For instance, the various tangible storage media **336** can interface with the bus **340** via storage medium interface **326.** Computer system **300** can have any suitable physical form, including but not limited to one or more integrated circuits (ICs), printed circuit boards (PCBs), mobile handheld devices (such as mobile telephones or PDAs), laptop or notebook computers, distributed computer systems, computing grids, or servers.

Computer system **300** includes one or more processor(s) **301** (e.g., central processing units (CPUs) or general purpose graphics processing units (GPGPUs)) that carry out functions. Processor(s) **301** optionally contains a cache memory unit **302** for temporary local storage of instructions, data, or computer addresses. Processor(s) **301** are configured to assist in execution of computer readable instructions. Computer system **300** can provide functionality for the depictions in **FIGs. 1-2** as a result of the processor(s) **301** executing non-transitory, processor-executable instructions embodied in one or more tangible computer-readable storage media, such as memory **303**, storage **308,** storage devices **335,** and/or storage medium **336.** The computer-readable media can store software that implements particular embodiments, and processor(s) **301** can execute the software. Memory **303** can read the software from one or more other computer-readable media (such as mass storage device(s) **335, 336)** or from one or more other sources through a suitable interface, such as network interface **320**. The software can cause processor(s) **301** to carry out one or more processes or one or more steps of one or more processes described or illustrated herein. Carrying out such processes or steps can include defining data structures stored in memory **303** and modifying the data structures as directed by the software.

The memory **303** can include various components (e.g., machine readable media) including, but not limited to, a random access memory component (e.g., RAM **304)** (e.g., static RAM (SRAM), dynamic RAM (DRAM), ferroelectric random access memory (FRAM), phase-change random access memory (PRAM), etc.), a read-only memory component (e.g., ROM **305),** and any combinations thereof. ROM **305** can act to communicate data and instructions unidirectionally to processor(s) **301,** and RAM **304** can act to communicate data and instructions bidirectionally with processor(s) **301.** ROM **305** and RAM **304** can include any suitable tangible computer-readable media described below. In one example, a basic input/output system **306** (BIOS), including basic routines that help to transfer information between elements within computer system **300**, such as during start-up, can be stored in the memory **303**.

Fixed storage **308** is connected bidirectionally to processor(s) **301,** optionally through storage control unit **307**. Fixed storage **308** provides additional data storage capacity and can also include any suitable tangible computer-readable media described herein. Storage **308** can be used to store operating system **309**, executable(s) **310,** data **311,** applications **312** (application programs), and the like. Storage **308** can also include an optical disk drive, a solid-state memory device (e.g., flash-based systems), or a combination of any of the above. Information in storage **308** may, in appropriate cases, be incorporated as virtual memory in memory **303**.

In one example, storage device(s) **335** can be removably interfaced with computer system **300** (e.g., via an external port connector (not shown)) via a storage device interface **325.** Particularly, storage device(s) **335** and an associated machine-readable medium can provide non-volatile and/or volatile storage of machine-readable instructions, data structures, program modules, and/or other data for the computer system **300.** In one example, software can reside, completely or partially, within a machine-readable medium on storage device(s) **335.** In another example, software can reside, completely or partially, within processor(s) **301.**

Bus **340** connects a wide variety of subsystems. Herein, reference to a bus can encompass one or more digital signal lines serving a common function, where appropriate. Bus **340** can be any of several types of bus structures including, but not limited to, a memory bus, a memory controller, a peripheral bus, a local bus, and any combinations thereof, using any of a variety of bus architectures. As an example and not by way of limitation, such architectures include an Industry Standard Architecture (ISA) bus, an Enhanced ISA (EISA) bus, a Micro Channel Architecture (MCA) bus, a Video Electronics Standards Association local bus (VLB), a Peripheral Component Interconnect (PCI) bus, a PCI-Express (PCI-X) bus, an Accelerated Graphics Port (AGP) bus, HyperTransport (HTX) bus, serial advanced technology attachment (SATA) bus, and any combinations thereof.

Computer system **300** can also include an input device **333.** In one example, a user of computer system **300** can enter commands and/or other information into computer system **300** via input device(s) **333.** Examples of an input device(s) **333** include, but are not limited to, an alpha-numeric input device (e.g., a keyboard), a pointing device (e.g., a mouse or touchpad), a touchpad, a touch screen, a multi-touch screen, a joystick, a stylus, a gamepad, an audio input device (e.g., a microphone, a voice response system, etc.), an optical scanner, a video or still image capture device (e.g., a camera), and any combinations thereof. In some embodiments, the input device is a Kinect, Leap Motion, or the like. Input device(s) **333** can be interfaced to bus **340** via any of a variety of input interfaces **323** (e.g., input interface **323)** including, but not limited to, serial, parallel, game port, USB, FIREWIRE, THUNDERBOLT, or any combination of the above.

In particular embodiments, when computer system **300** is connected to network **330**, computer system **300** can communicate with other devices, specifically mobile devices and enterprise systems, distributed computing systems, cloud storage systems, cloud computing systems, and the like, connected to network **330.** Communications to and from computer system **300** can be sent through network interface **320**. For example, network interface **320** can receive incoming communications (such as requests or responses from other devices) in the form of one or more packets (such as Internet Protocol (IP) packets) from network **330**, and computer system **300** can store the incoming communications in memory **303** for processing. Computer system **300** can similarly store outgoing communications (such as requests or responses to other devices) in the form of one or more packets in memory **303** and communicated to network **330** from network interface **320**. Processor(s) **301** can access these communication packets stored in memory **303** for processing.

Examples of the network interface **320** include, but are not limited to, a network interface card, a modem, and any combination thereof. Examples of a network **330** or network segment **330** include, but are not limited to, a distributed computing system, a cloud computing system, a wide area network (WAN) (e.g., the Internet, an enterprise network), a local area network (LAN) (e.g., a network associated with an office, a building, a campus or other relatively small geographic space), a telephone network, a direct connection between two computing devices, a peer-to-peer network, and any combinations thereof. A network, such as network **330**, can employ a wired and/or a wireless mode of communication. In general, any network topology can be used.

Information and data can be displayed through a display **332.** Examples of a display **332** include, but are not limited to, a cathode ray tube (CRT), a liquid crystal display (LCD), a thin film transistor liquid crystal display (TFT-LCD), an organic liquid crystal display (OLED) such as a passive-matrix OLED (PMOLED) or active-matrix OLED (AMOLED) display, a plasma display, and any combinations thereof. The display **332** can interface to the processor(s) **301,** memory **303**, and fixed storage **308**, as well as other devices, such as input device(s) **333,** via the bus **340.** The display **332** is linked to the bus **340** via a video interface **322,** and transport of data between the display **332** and the bus **340** can be controlled via the graphics control **321.** In some embodiments, the display is a video projector. In some embodiments, the display is a head-mounted display (HMD) such as a VR headset. In further embodiments, suitable VR headsets include, by way of non-limiting examples, HTC Vive, Oculus Rift, Samsung Gear VR, Microsoft HoloLens, Razer OSVR, FOVE VR, Zeiss VR One, Avegant Glyph, Freefly VR headset, and the like. In still further embodiments, the display is a combination of devices such as those disclosed herein.

In addition to a display **332,** computer system **300** can include one or more other peripheral output devices **334** including, but not limited to, an audio speaker, a printer, a storage device, and any combinations thereof. Such peripheral output devices can be connected to the bus **340** via an output interface **324.** Examples of an output interface **324** include, but are not limited to, a serial port, a parallel connection, a USB port, a FIREWIRE port, a THUNDERBOLT port, and any combinations thereof.

In addition, or as an alternative, computer system **300** can provide functionality as a result of logic hardwired or otherwise embodied in a circuit, which can operate in place of or together with software to execute one or more processes or one or more steps of one or more processes described or illustrated herein. Reference to software in this disclosure can encompass logic, and reference to logic can encompass software. Moreover, reference to a computer-readable medium can encompass a circuit (such as an IC) storing software for execution, a circuit embodying logic for execution, or both, where appropriate. The present disclosure encompasses any suitable combination of hardware, software, or both.

Those of skill in the art will appreciate that the various illustrative logical blocks, modules, circuits, and algorithm steps described in connection with the embodiments disclosed herein can be implemented as electronic hardware, computer software, or combinations of both. To clearly illustrate this interchangeability of hardware and software, various illustrative components, blocks, modules, circuits, and steps have been described above generally in terms of their functionality.

The various illustrative logical blocks, modules, and circuits described in connection with the embodiments disclosed herein can be implemented or performed with a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor can be a microprocessor, but in the alternative, the processor can be any conventional processor, controller, microcontroller, or state machine. A processor can also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein can be embodied directly in hardware, in a software module executed by one or more processor(s), or in a combination of the two. A software module can reside in RAM memory, flash memory, ROM memory, EPROM memory, EEPROM memory, registers, hard disk, a removable disk, a CD-ROM, or any other form of storage medium known in the art. An exemplary storage medium is coupled to the processor such the processor can read information from, and write information to, the storage medium. In the alternative, the storage medium can be integral to the processor. The processor and the storage medium can reside in an ASIC. The ASIC can reside in a user terminal. In the alternative, the processor and the storage medium can reside as discrete components in a user terminal.

In accordance with the description herein, suitable computing devices include, by way of non-limiting examples, server computers, desktop computers, laptop computers, notebook computers, sub-notebook computers, netbook computers, netpad computers, set-top computers, media streaming devices, handheld computers, Internet appliances, mobile smartphones, tablet computers, personal digital assistants, video game consoles, and vehicles. Those of skill in the art will also recognize that select televisions, video players, and digital music players with optional computer network connectivity are suitable for use in the system described herein. Suitable tablet computers, in various embodiments, include those with booklet, slate, and convertible configurations, known to those of skill in the art.

In some embodiments, the computing device includes an operating system configured to perform executable instructions. The operating system is, for example, software, including programs and data, which manages the device's hardware and provides services for execution of applications. Those of skill in the art will recognize that suitable server operating systems include, by way of non-limiting examples, FreeBSD, OpenBSD, NetBSD^{®}, Linux, Apple^{®} Mac OS X Server^{®}, Oracle^{®} Solaris^{®}, Windows Server^{®}, and Novell^{®} NetWare^{®}. Those of skill in the art will recognize that suitable personal computer operating systems include, by way of non-limiting examples, Microsoft^{®} Windows^{®}, Apple^{®} Mac OS X^{®}, UNIX^{®}, and UNIX-like operating systems such as GNU/Linux^{®}. In some embodiments, the operating system is provided by cloud computing. Those of skill in the art will also recognize that suitable mobile smartphone operating systems include, by way of non-limiting examples, Nokia^{®} Symbian^{®} OS, Apple^{®} iOS^{®}, Research In Motion^{®} BlackBerry OS^{®}, Google^{®} Android^{®}, Microsoft^{®} Windows Phone^{®} OS, Microsoft^{®} Windows Mobile^{®} OS, Linux^{®}, and Palm^{®} WebOS^{®}. Those of skill in the art will also recognize that suitable media streaming device operating systems include, by way of non-limiting examples, Apple TV^{®}, Roku^{®}, Boxee^{®}, Google TV^{®}, Google Chromecast^{®}, Amazon Fire^{®}, and Samsung^{®} HomeSync^{®}. Those of skill in the art will also recognize that suitable video game console operating systems include, by way of non-limiting examples, Sony^{®} PS3^{®}, Sony^{®} PS4^{®}, Microsoft^{®} Xbox 360^{®}, Microsoft Xbox One, Nintendo^{®} Wii^{®}, Nintendo^{®} Wii U^{®}, and Ouya^{®}.

### Non-transitory computer readable storage medium

In some embodiments, the Vagus nerve stimulation devices, systems, and methods disclosed herein include one or more non-transitory computer readable storage media encoded with a program including instructions executable by the operating system of an optionally networked computing device. In further embodiments, a computer readable storage medium is a tangible component of a computing device. In still further embodiments, a computer readable storage medium is optionally removable from a computing device. In some embodiments, a computer readable storage medium includes, by way of non-limiting examples, CD-ROMs, DVDs, flash memory devices, solid state memory, magnetic disk drives, magnetic tape drives, optical disk drives, distributed computing systems including cloud computing systems and services, and the like. In some cases, the program and instructions are permanently, substantially permanently, semi-permanently, or non-transitorily encoded on the media.

### Computer program

In some embodiments, the Vagus nerve stimulation devices, systems, and methods disclosed herein include at least one computer program, or use of the same. A computer program includes a sequence of instructions, executable by one or more processor(s) of the computing device's CPU, written to perform a specified task. Computer readable instructions can be implemented as program modules, such as functions, objects, Application Programming Interfaces (APIs), computing data structures, and the like, that perform particular tasks or implement particular abstract data types. In light of the disclosure provided herein, those of skill in the art will recognize that a computer program can be written in various versions of various languages.

The functionality of the computer readable instructions can be combined or distributed as desired in various environments. In some embodiments, a computer program comprises one sequence of instructions. In some embodiments, a computer program comprises a plurality of sequences of instructions. In some embodiments, a computer program is provided from one location. In other embodiments, a computer program is provided from a plurality of locations. In various embodiments, a computer program includes one or more software modules. In various embodiments, a computer program includes, in part or in whole, one or more web applications, one or more mobile applications, one or more standalone applications, one or more web browser plug-ins, extensions, add-ins, or add-ons, or combinations thereof.

In some embodiments, a computer program includes a web application for implementation of the Vagus nerve stimulation devices, systems, and methods as described herein. In light of the disclosure provided herein, those of skill in the art will recognize that a web application, in various embodiments, utilizes one or more software frameworks and one or more database systems. In some embodiments, a web application is created upon a software framework such as Microsoft^{®} .NET or Ruby on Rails (RoR). In some embodiments, a web application utilizes one or more database systems including, by way of non-limiting examples, relational, non-relational, object oriented, associative, and XML database systems. In further embodiments, suitable relational database systems include, by way of non-limiting examples, Microsoft^{®} SQL Server, mySQL^{™}, and Oracle^{®}. Those of skill in the art will also recognize that a web application, in various embodiments, is written in one or more versions of one or more languages. A web application can be written in one or more markup languages, presentation definition languages, client-side scripting languages, server-side coding languages, database query languages, or combinations thereof. In some embodiments, a web application is written to some extent in a markup language such as Hypertext Markup Language (HTML), Extensible Hypertext Markup Language (XHTML), or eXtensible Markup Language (XML). In some embodiments, a web application is written to some extent in a presentation definition language such as Cascading Style Sheets (CSS). In some embodiments, a web application is written to some extent in a client-side scripting language such as Asynchronous Javascript and XML (AJAX), Flash^{®} Actionscript, Javascript, or Silverlight^{®}. In some embodiments, a web application is written to some extent in a server-side coding language such as Active Server Pages (ASP), ColdFusion^{®}, Perl, Java^{™}, JavaServer Pages (JSP), Hypertext Preprocessor (PHP), Python^{™}, Ruby, Tcl, Smalltalk, WebDNA^{®}, or Groovy. In some embodiments, a web application is written to some extent in a database query language such as Structured Query Language (SQL). In some embodiments, a web application integrates enterprise server products such as IBM^{®} Lotus Domino^{®}. In some embodiments, a web application includes a media player element. In various further embodiments, a media player element utilizes one or more of many suitable multimedia technologies including, by way of non-limiting examples, Adobe^{®} Flash^{®}, HTML 5, Apple^{®} QuickTime^{®}, Microsoft^{®} Silverlight^{®}, Java^{™}, and Unity^{®}.

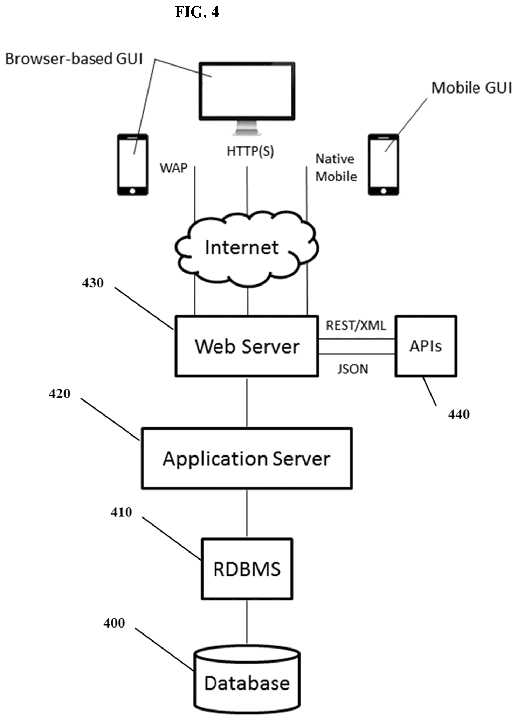

Referring to **FIG. 4****,** in a particular embodiment, an application provision system comprises one or more databases **400** accessed by a relational database management system (RDBMS) **410.** Suitable RDBMSs include Firebird, MySQL, PostgreSQL, SQLite, Oracle Database, Microsoft SQL Server, IBM DB2, IBM Informix, SAP Sybase, SAP Sybase, Teradata, and the like. In this embodiment, the application provision system further comprises one or more application severs **420** (such as Java servers, .NET servers, PHP servers, and the like) and one or more web servers **430** (such as Apache, IIS, GWS and the like). The web server(s) optionally expose one or more web services via app application programming interfaces (APIs) **440.** Via a network, such as the Internet, the system provides browser-based and/or mobile native user interfaces.

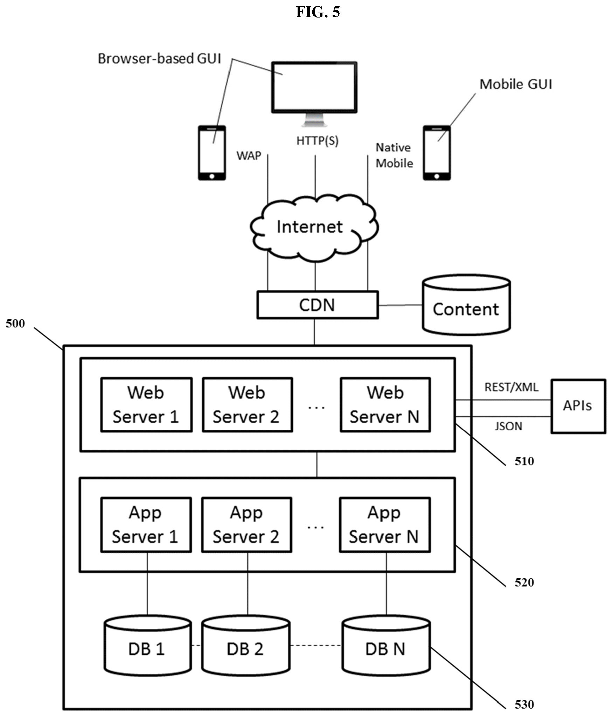

Referring to **FIG. 5****,** in a particular embodiment, an application provision system alternatively has a distributed, cloud-based architecture **500** and comprises elastically load balanced, auto-scaling web server resources **510** and application server resources **520** as well synchronously replicated databases **530**.

In some embodiments, a computer program includes a mobile application provided to a mobile computing device for implementation of the Vagus nerve stimulation devices, systems, and methods as described herein. In some embodiments, the mobile application is provided to a mobile computing device at the time it is manufactured. In other embodiments, the mobile application is provided to a mobile computing device via the computer network described herein.

In view of the disclosure provided herein, a mobile application is created by techniques known to those of skill in the art using hardware, languages, and development environments known to the art. Those of skill in the art will recognize that mobile applications are written in several languages. Suitable programming languages include, by way of non-limiting examples, C, C++, C#, Objective-C, Java^{™}, Javascript, Pascal, Object Pascal, Python^{™}, Ruby, VB.NET, WML, and XHTML/HTML with or without CSS, or combinations thereof.

Suitable mobile application development environments are available from several sources. Commercially available development environments include, by way of non-limiting examples, AirplaySDK, alcheMo, Appcelerator^{®}, Celsius, Bedrock, Flash Lite, .NET Compact Framework, Rhomobile, and WorkLight Mobile Platform. Other development environments are available without cost including, by way of non-limiting examples, Lazarus, MobiFlex, MoSync, and Phonegap. Also, mobile device manufacturers distribute software developer kits including, by way of non-limiting examples, iPhone and iPad (iOS) SDK, Android^{™} SDK, BlackBerry^{®} SDK, BREW SDK, Palm^{®} OS SDK, Symbian SDK, webOS SDK, and Windows^{®} Mobile SDK.

Those of skill in the art will recognize that several commercial forums are available for distribution of mobile applications including, by way of non-limiting examples, Apple^{®} App Store, Google^{®} Play, Chrome WebStore, BlackBerry^{®} App World, App Store for Palm devices, App Catalog for webOS, Windows^{®} Marketplace for Mobile, Ovi Store for Nokia^{®} devices, Samsung^{®} Apps, and Nintendo^{®} DSi Shop.

In some embodiments, a computer program includes a standalone application, which is a program that is run as an independent computer process, not an add-on to an existing process, e.g., not a plug-in for implementation of the Vagus nerve stimulation devices, systems, and methods as described herein. Those of skill in the art will recognize that standalone applications are often compiled. A compiler is a computer program(s) that transforms source code written in a programming language into binary object code such as assembly language or machine code. Suitable compiled programming languages include, by way of non-limiting examples, C, C++, Objective-C, COBOL, Delphi, Eiffel, Java^{™}, Lisp, Python^{™}, Visual Basic, and VB .NET, or combinations thereof. Compilation is often performed, at least in part, to create an executable program. In some embodiments, a computer program includes one or more executable complied applications.

In some embodiments, the computer program includes a web browser plug-in (e.g., extension, etc.) for implementation of the Vagus nerve stimulation devices, systems, and methods as described herein. In computing, a plug-in is one or more software components that add specific functionality to a larger software application. Makers of software applications support plug-ins to enable third-party developers to create abilities which extend an application, to support easily adding new features, and to reduce the size of an application. When supported, plug-ins enable customizing the functionality of a software application. For example, plug-ins are commonly used in web browsers to play video, generate interactivity, scan for viruses, and display particular file types. Those of skill in the art will be familiar with several web browser plug-ins including, Adobe^{®} Flash^{®} Player, Microsoft^{®} Silverlight^{®}, and Apple^{®} QuickTime^{®}. In some embodiments, the toolbar comprises one or more web browser extensions, add-ins, or add-ons. In some embodiments, the toolbar comprises one or more explorer bars, tool bands, or desk bands.

In view of the disclosure provided herein, those of skill in the art will recognize that several plug-in frameworks are available that enable development of plug-ins in various programming languages, including, by way of non-limiting examples, C++, Delphi, Java^{™}, PHP, Python^{™}, and VB .NET, or combinations thereof.

Web browsers (also called Internet browsers) are software applications, designed for use with network-connected computing devices, for retrieving, presenting, and traversing information resources on the World Wide Web. Suitable web browsers include, by way of non-limiting examples, Microsoft^{®} Internet Explorer^{®}, Mozilla^{®} Firefox^{®}, Google^{®} Chrome, Apple^{®} Safari^{®}, Opera Software^{®} Opera^{®}, and KDE Konqueror. In some embodiments, the web browser is a mobile web browser. Mobile web browsers (also called microbrowsers, mini-browsers, and wireless browsers) are designed for use on mobile computing devices including, by way of non-limiting examples, handheld computers, tablet computers, netbook computers, subnotebook computers, smartphones, music players, personal digital assistants (PDAs), and handheld video game systems. Suitable mobile web browsers include, by way of non-limiting examples, Google^{®} Android^{®} browser, RIM BlackBerry^{®} Browser, Apple^{®} Safari^{®}, Palm^{®} Blazer, Palm^{®} WebOS^{®} Browser, Mozilla^{®} Firefox^{®} for mobile, Microsoft^{®} Internet Explorer^{®} Mobile, Amazon^{®} Kindle^{®} Basic Web, Nokia^{®} Browser, Opera Software^{®} Opera^{®} Mobile, and Sony^{®} PSP^{™} browser.

### Software Modules

In some embodiments, the Vagus nerve stimulation devices, systems, and methods disclosed herein include software, server, and/or database modules, or use of the same. In view of the disclosure provided herein, software modules are created by techniques known to those of skill in the art using machines, software, and languages known to the art. The software modules disclosed herein are implemented in a multitude of ways. In some cases, the software modules described herein can at least partially be the learning engine.

In various embodiments, a software module comprises a file, a section of code, a programming object, a programming structure, or combinations thereof. In further various embodiments, a software module comprises a plurality of files, a plurality of sections of code, a plurality of programming objects, a plurality of programming structures, or combinations thereof. In various embodiments, the one or more software modules comprise, by way of non-limiting examples, a web application, a mobile application, and a standalone application. In some embodiments, software modules are in one computer program or application. In other embodiments, software modules are in more than one computer program or application. In some embodiments, software modules are hosted on one machine. In other embodiments, software modules are hosted on more than one machine. In further embodiments, software modules are hosted on a distributed computing platform such as a cloud computing platform. In some embodiments, software modules are hosted on one or more machines in one location. In other embodiments, software modules are hosted on one or more machines in more than one location.

### Methods Utilizing a Computer

The Vagus nerve stimulation devices, systems, and methods described herein can utilize one or more computers. The computer can be used for managing customer and sample information such as sample or customer tracking, database management, analyzing molecular profiling data, analyzing cytological data, storing data, billing, marketing, reporting results, storing results, or a combination thereof. The computer can include a monitor or other graphical interface for displaying data, results, billing information, marketing information (e.g. demographics), customer information, or sample information. The computer can also include means for data or information input. The computer can include a processing unit and fixed or removable media or a combination thereof. The computer can be accessed by a user in physical proximity to the computer, for example via a keyboard and/or mouse, or by a user that does not necessarily have access to the physical computer through a communication medium such as a modem, an internet connection, a telephone connection, or a wired or wireless communication signal carrier wave. In some cases, the computer can be connected to a server or other communication device for relaying information from a user to the computer or from the computer to a user. In some cases, the user can store data or information obtained from the computer through a communication medium on media, such as removable media. It is envisioned that data relating to the methods can be transmitted over such networks or connections for reception and/or review by a party. The receiving party can be but is not limited to an individual, a health care provider or a health care manager. In one instance, a computer-readable medium includes a medium suitable for transmission of a result of an analysis of a biological sample. The medium can include a result of a subject, wherein such a result is derived using the methods described herein.

The entity obtaining the sample information can enter it into a database for the purpose of one or more of the following: inventory tracking, assay result tracking, order tracking, customer management, customer service, billing, and sales. Sample information can include, but is not limited to: customer name, unique customer identification, customer associated medical professional, indicated assay or assays, assay results, adequacy status, indicated adequacy tests, medical history of the individual, preliminary diagnosis, suspected diagnosis, sample history, insurance provider, medical provider, third party testing center or any information suitable for storage in a database. Sample history can include but is not limited to: age of the sample, type of sample, method of acquisition, method of storage, or method of transport.

The database can be accessible by a customer, medical professional, insurance provider, or other third party. Database access can take the form of digital processing communication such as a computer or telephone. The database can be accessed through an intermediary such as a customer service representative, business representative, consultant, independent testing center, or medical professional. The availability or degree of database access or sample information, such as assay results, can change upon payment of a fee for products and services rendered or to be rendered. The degree of database access or sample information can be restricted to comply with generally accepted or legal requirements for patient or customer confidentiality.

### EXAMPLES

The following illustrative examples are representative of embodiments of the stimulation, systems, and methods described herein and are not meant to be limiting in any way.

### Example 1. Vagus Nerve Stimulation for Migraine Relief

Multiple subjects suffering from acute migraine headaches are fitted with a stimulation device described herein, where two electrodes are positioned to contact the left cymba concha of the subject. Each subject is given an electrical stimulation through the device for a period of 5 minutes, where the stimulus provides a stimulation at 200 Hz, with a pulse width of 20 milliseconds and a square waveform, with an applied current at the tolerability level for each subject, e.g., between about 0.25 and about 2 milliAmps. Each subject reports significant reduction or elimination of migraine pain within 1 to 6 hours following stimulation.

### Example 2. Vagus Nerve Stimulation for Treating Arthritis

Multiple subjects suffering from acute rheumatoid arthritis are administered a personalized stimulation protocol once per day, three days per week, for one month. Following completion of the full administration protocol, a statistically significant improvement in the arthritis symptoms of the subject is shown.

While the foregoing disclosure has been described in some detail for purposes of clarity and understanding, it will be clear to one skilled in the art from a reading of this disclosure that various changes in form and detail can be made without departing from the true scope of the disclosure. For example, all the techniques and apparatus described above can be used in various combinations. All publications, patents, patent applications, and/or other documents cited in this application are incorporated by reference in their entirety for all purposes to the same extent as if each individual publication, patent, patent application, and/or other document were individually and separately indicated to be incorporated by reference for all purposes.

## Claims

1. A therapeutic system, comprising:
a housing structure configured to be positioned over, adjacent to, or within an ear of a subject;
an auricular stimulation device (102) integrated into the housing structure, the auricular stimulation device (102) comprising at least a first and at least a second electrode (108, 110) positioned spaced apart from each other and configured to be positioned proximal to the subject's ear when the housing structure is positioned over, within, or adjacent to the ear of the subject, the proximal positioning configured to deliver electrical stimulation to a Vagus nerve of the subject;
a controller (114), coupled to the stimulation device (102) and configured to control and/or modulate application of electrical stimulation delivered by the stimulation device in response to one or more input parameters;
a monitoring system (116) operatively coupled to the controller (114), the monitoring system (116) configured to provide an assessment of the subject, the assessment to provide the one or more input parameters to the controller; and
a learning engine operatively coupled to one or more of the monitoring system (116) and the controller (114), wherein the learning engine is programmed to aggregate assessment information and determine one or more optimal input parameters or assessment criteria configured to be applied to the controller as input to the stimulation device.

2. The system of claim 1, further comprising a computer system to selectively modify one or more electrical stimulation parameters and apply the electrical stimulation in real-time.

3. The system of claim 1, further comprising the controller (114) to deliver audio content to an audio delivery system, wherein the delivered audio content is selected to augment or increase a physiological impact of the electrical stimulation delivered by the stimulation device.

4. The system of claim 1, further comprising a stimulation normalization system configured to ensure delivery of a consistent stimulation to different subjects or the same subject at different times.

5. The system of claim 4, wherein the stimulation system measures impedance between the first and second electrodes positioned relative to the subject, and modulates stimulation based upon the measured impedance.

6. The system of claim 1, wherein the housing structure is a wearable device, and further comprising housing or mounting the controller within the housing structure.

7. The system of claim 6, wherein the wearable device includes a headset, an ear-phone, an ear cuff, a headphone, or a similar structure.

8. The system of claim 6, wherein the wearable device is configured to position the first and second electrodes in electrical contact with an exterior ear structure of the subject.

9. The system of claim 1, wherein the first and second electrodes each have a conical shape configured to provide targeted contact within the ear of the subject.

10. The system of claim 1, further comprising a non-conductive material provided over a tip of one or more of the first and second electrodes, the non-conductive material configured to be saturated with a conductive medium.

11. The system of claim 1, further comprising a conductive polymer tip configured to be placed over a tip of the first and/or second electrode.

12. The system of claim 1, wherein the electrical stimulation is provided to an auricular branch of the Vagus nerve.
